# EUROPEAN PATENT SPECIFICATION

(11) **EP 2 361 575 B1**
(45) Date of publication and mention of the grant of the patent: **13.03.2013**
(21) Application number: 10188019.3
(22) Date of filing: 19.10.2010
(51) Int. Cl.: A61B 17/88, A61B 17/56, A61B 17/34, A61B 17/00, A61B 17/06

(54) **Introducer device for use in nuss procedure**
Einführungsvorrichtung zur Verwendung in einem Verfahren nach Nuss
Dispositif introducteur pour une utilisation dans une procédure NUSS

(30) Priority: 23.02.2010 TW 099105153
(43) Date of publication of application: 31.08.2011
(73) Proprietor: Chi Mei Medical Center, Tainan (TW)
(72) Inventor: Su, Ying-Chieh, Tainan City, Beimen Rd. (TW)
(74) Representative: Cohausz & Florack

(56) References cited:
- WO-A1-2005/055844
- WO-A1-2008/114915
- CN-Y- 201 275 102
- US-A- 6 024 759

## Description

The invention relates to an introducer device for use in Nuss procedure to guide a concave steel bar in retrieving an insertion route which passes through and between the sternum/ribs and the heart/lungs of a patient, and which is curved away from the sternum/ribs, the concave steel bar having a curved middle portion which is configured to fit in the insertion route, and which has a forward-facing surface and a rearward-facing surface that are concave and convex respectively, said introducer device comprising an elongated introducer body which extends in a lengthwise direction to terminate at a leading tip end that is adapted to be coupled to the concave steel bar for retrieving the insertion route, and a grip end that is opposite to said leading tip, and which has a curve segment disposed proximate to said leading tip end, and configured to establish the insertion route when said leading tip end is led to pass through and between the sternum/ribs and the heart/lungs subsequent to being introduced through a first incision made at a lateral side of the patient's chest, said curve segment having a sternum-side surface and a heart-side surface that are concave and convex respectively. Such an introducer device is for example known from WO 2008/114915 A1 and US 6,024,759.

Pectus excavatum, also known as funnel chest, is a congenital deformity in the chest, and is the result of abnormal growth of sternum and ribs. This deformity causes a sunken appearance in the anterior wall of the chest, i.e., the distance between the sternum and the spine is reduced as compared to a normal chest. Depending on the severity, pectus excavatum may be an appearance problem, or may impair cardiac and respiratory functions.

One of the most common ways for treating pectus excavatum is called Nuss procedure, which involves putting a steel bar 100 with a predetermined curvature (also referred to as a Nuss bar) into the patient' s chest 200 between the heart 202 /lungs (not shown) and the sternum 201 / ribs (not shown) for pushing outward the patient's sternum 201.

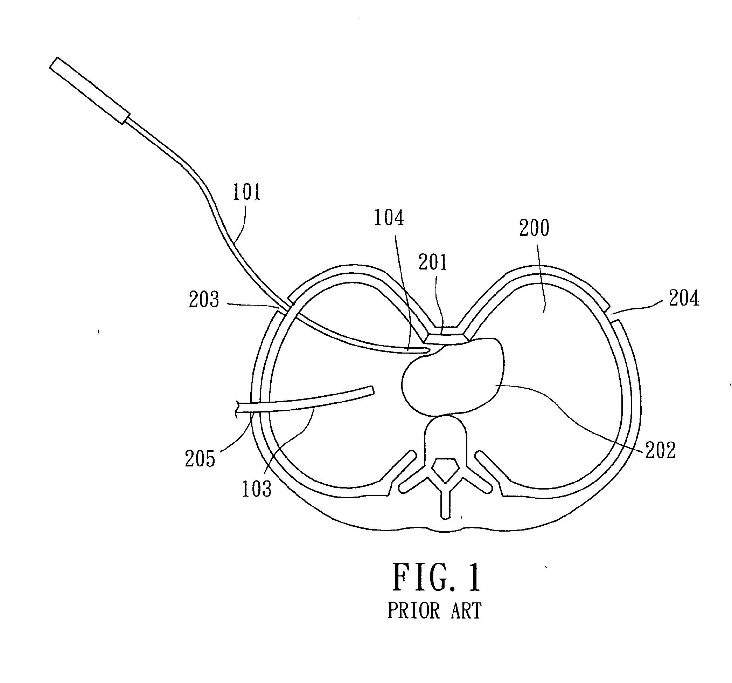

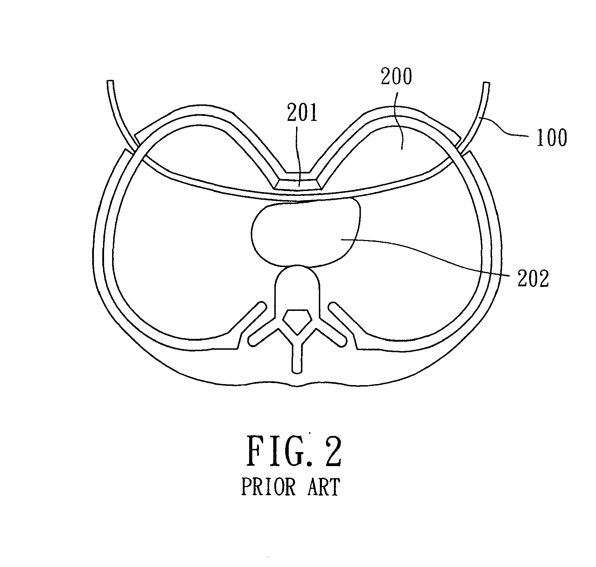

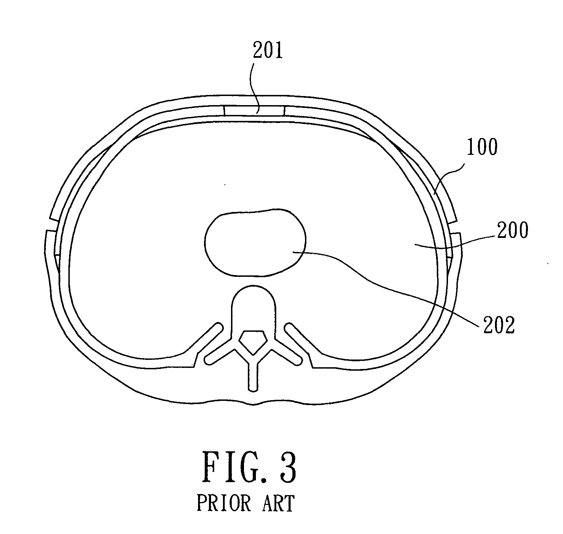

As shown in Figure 1, in order to put the steel bar 100 into the patient's chest 200, a curved introducer 101 is first inserted into the patent's chest 200 via a first incision 203 made below the right armpit for continuing on squeezing through and between the sternum 201 and the heart 202. Subsequent to establishment of an insertion route between the sternum 201 and the heart 202, a leading tip end 104 of the introducer 101 is led to exit a second incision 204 made below the left armpit and out of the patient's chest 200. Thereafter, the steel bar 100 is coupled to the leading tip end 104 of the introducer 101 and retrieves the insertion route that the introducer 101 has just passed in order to be guided through and between the sternum 201 and the heart 202. Lastly, after the introducer 201 has completely exited the patient's chest 200 and is uncoupled from the steel bar 100 (as shown in Figure 2), the steel bar 100 is flipped to a convex position so as to push outward the sternum 201 (as shown in Figure 3) to correct the deformity.

Although the Nuss procedure is minimally invasive, since the sternum 201 is normally very close to, or even rests tightly against the heart 202 of a patient with pectus excavatum, there still lies the risk of abrading or puncturing the heart 202 and lungs (not shown) of the patient. Therefore, to assist insertion of the introducer 101 into the patient's chest 200 and to minimize the risks of damaging the heart 202 and the lungs, a thoracoscope 103 is often inserted into the patient's chest 200 through another incision 205 made at the right lateral side of the patient's chest 200. However, due to the limited space between the sternum 201 and the heart 202, the thoracoscope 103 cannot be positioned further but to trail behind the leading tip end 104 in the right side of the thoracic cavity 200. Therefore, once the leading tip end 104 of the introducer 101 is led to pass between the sternum 201 and the heart 202, the thoracoscope 103 can no longer provide an effective instant visualization to a surgeon for keeping eye on the leading tip end 104 unless the thoracoscope 103 is otherwise displaced into the left side of the thoracic cavity 200. In addition, since radiography and computerized tomography (CT) scan cannot be performed during surgery, the surgeon's clinical experience is heavily relied upon under such circumstance, and it might be time-consuming even for an experienced surgeon. Moreover, there is still the possibility of making a wrong judgment when no other technological assistance can be provided to the surgeon. This greatly increases a patient's risks during surgery, and lengthens operation time.

Therefore, the object of the present invention is to provide an introducer device capable of facilitating diagnostic judgment during Nuss procedure.

To achieve the above object there is provided an introducer device with the features of the preamble of claim 1, characterized by the features of the characterizing portion of claim 1. Embodiments are defined in the subclaims.

According to the present invention, there is provided an introducer device for use in Nuss procedure to guide a concave steel bar in retrieving an insertion route which passes through and between the sternum/ribs and the heart/lungs of a patient, and which is curved away from the sternum/ribs. The concave steel bar has a curved middle portion which is configured to fit in the insertion route, and which has a forward-facing surface and a rearward-facing surface that are concave and convex respectively.

The introducer device includes an elongated introducer body and an ultrasonic probe. The elongated introducer body extends in a lengthwise direction to terminate at a leading tip end that is adapted to be coupled to the concave steel bar for retrieving the insertion route, and a grip end that is opposite to the leading tip, and has a curve segment disposed proximate to the leading tip end, and configured to establish the insertion route when the leading tip end is led to pass through and between the sternum/ribs and the heart/lungs subsequent to being introduced through a first incision made at a lateral side of the patient's chest. The curve segment has a sternum-side surface and a heart-side surface that are concave and convex respectively. The ultrasonic probe is coupled to be moved with the leading tip end so as to transmit ultrasonic waves towards the heart/lungs of the patient and to receive reflected ultrasonic waves traveling from the heart/lungs when passing through and between the sternum/ribs and the heart/lungs.

The advantages and effects of the present invention lie in that through the provision of the ultrasonic probe, the introducer device of the present invention facilitate the surgeon to move the introducer body forward so as to pass through and between the heart and the sternum, thereby reducing the risks of abrading and puncturing the heart.

Other features and advantages of the present invention will become apparent in the following detailed description of the preferred embodiments with reference to the accompanying drawings, in which:
Figure 1 is a schematic sectional view of a patient's chest, illustrating insertion of a conventional introducer into the patient's chest during Nuss procedure;
Figure 2 is a schematic view similar to Figure 1, illustrating a steel bar extending through the patient' s chest;
Figure 3 is a schematic view similar to Figure 2, illustrating the steel bar flipped to push outward a sternum in the patient's chest;
Figure 4 is a perspective view of the preferred embodiment of an introducer device according to the present invention;
Figure 5 is a schematic view of a patient's chest, illustrating the introducer device passing through and between heart and sternum in the patient's chest during Nuss procedure;
Figure 6 is a schematic view similar to Figure 5, illustrating coupling of a concave steel bar with the introducer device for retrieving an insertion route established by the introducer device; and
Figure 7 is a schematic front view of the patient's chest, illustrating the introducer device extending through the patient's chest before guiding the concave steel bar to retrieve the insertion route.

It should be noted herein that the relative proportions and dimensions of the elements shown in the drawings are for illustrative purposes only, and should not be conceived to limit the scope of the present invention.

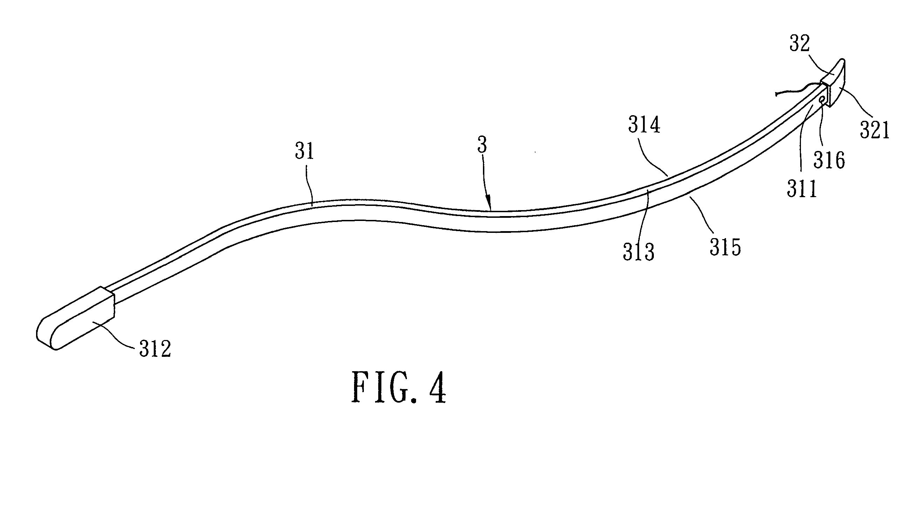

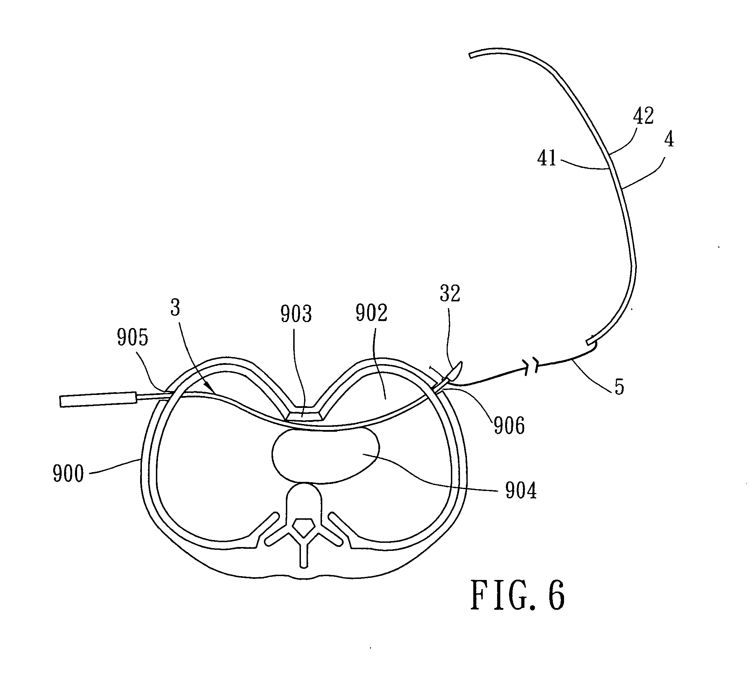

With reference to Figure 4, the preferred embodiment of an introducer device 3 is for use in Nuss procedure to guide a concave steel bar 4 (shown in Figure 6) in retrieving an insertion route which passes through and between the sternum 903 / ribs (not shown) and the heart 904 / lungs (not shown) of a patient 900, and which is curved away from the sternum 903 / ribs. The concave steel bar 4 has a curved middle portion which is configured to fit in the insertion route, and which has a forward-facing surface 41 and a rearward-facing surface 42 that are concave and convex respectively. The introducer device 3 includes an elongated introducer body 31 and an ultrasonic probe 32.

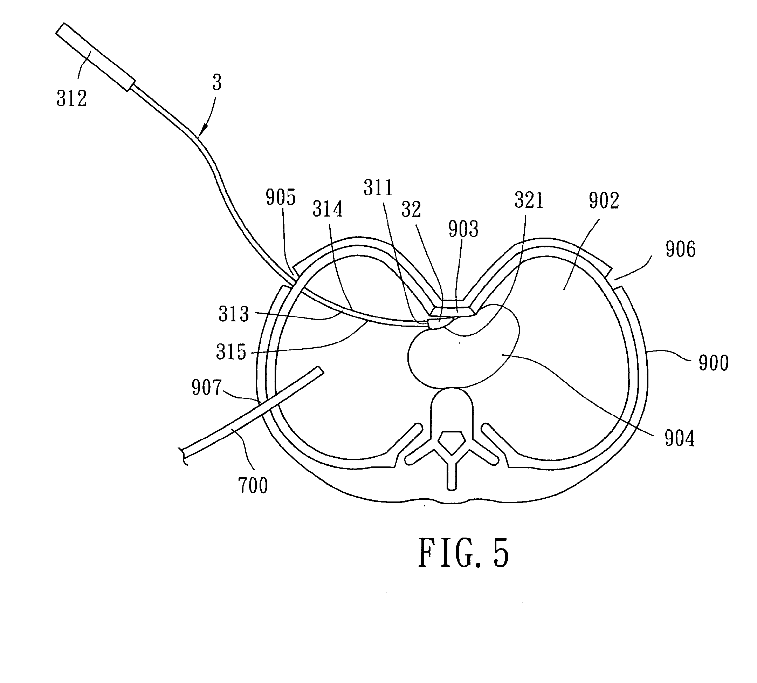

The elongated introducer body 31 extends in a lengthwise direction to terminate at a leading tip end 311 that is adapted to be coupled to the concave steel bar 4 for retrieving the insertion route, and a grip end 312 that is opposite to the leading tip 311. With further reference to Figure 5, the elongated introducer body 31 has a curve segment 313 disposed proximate to the leading tip end 311, and configured to establish the insertion route when the leading tip end 311 is led to pass through and between the sternum 903 / ribs and the heart 904 / lungs subsequent to being introduced through a first incision 905 made at the right lateral side of the patient's chest 902. The curve segment 313 has a sternum-side surface 314 and a heart-side surface 315 that are concave and convex, respectively.

In this embodiment, the leading tip end 311 of the elongated introducer body 31 is formed with a through hole 316 that is adapted to be attached to a string 5 connecting the concave steel bar 4 after the leading tip end 311 is led to exit a second incision 906 made at the left lateral side of the patient's chest 902 so as to enable the concave steel bar 4 to pass through and between the heart 904 / lungs and the sternum 903 / ribs as the elongated introducer body 31 retrieves the insertion route.

The ultrasonic probe 32 is coupled to be moved with the leading tip end 311 so as to transmit ultrasonic waves towards the heart 904 / lungs of the patient 900 and to receive reflected ultrasonic waves traveling from the heart 904 / lungs when passing through and between the sternum 903 / ribs and the heart 904 / lungs.

In this embodiment, the ultrasonic probe 32 has a probe surface 321 which is oriented to face the heart 904 / lungs, and which extends in the lengthwise direction from the heart-side surface 315 of the curve segment 313.

The ultrasonic probe 32 is adapted to be connected to an ultrasonic imaging device (not shown) so as to generate an ultrasonic image in connection with the ultrasonic waves received as a result of the traveling of the reflected ultrasonic waves from the heart 904 / lungs of the patient 902.

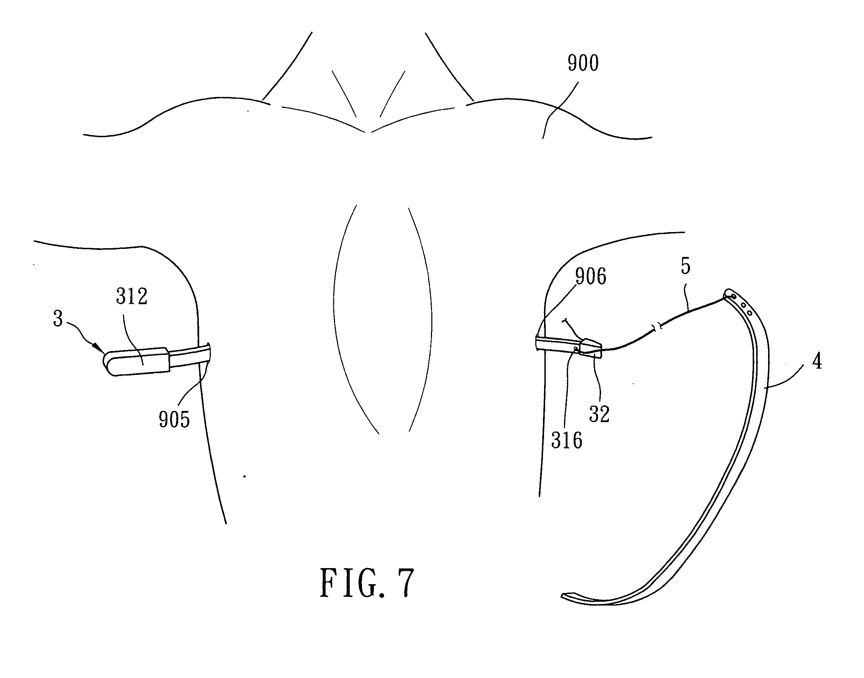

With reference to Figures 5, 6 and 7, during Nuss procedure, the leading tip end 311 of the elongated introducer body 31 is first introduced into the patient's chest 902 through the first incision 905 made at the right lateral side of the patient's chest 902. Then the ultrasonic probe 32 is activated so as to transmit and receive the ultrasonic waves. Prior to contacting any tissue or organs, the ultrasonic image generated by the ultrasonic imaging device that is connected to the ultrasonic probe 32 would be all black. Therefore, the aid of a thoracoscope 700 is still needed. The thoracoscope 700 is inserted into the patient's chest 902 through another incision 907 made at the right lateral side of the patient's chest 905, and facilitates the surgeon to move the leading tip end 311 of the elongated introducer body 31 forward until the leading tip end 311 is about to pass between the sternum 903 and the heart 904. At this time, the thoracoscope 700 can no longer be used to view the leading tip end 311 since the heart 904 blocks the view.

Once the probe surface 321 of the ultrasonic probe 32 contacts the heart 904 / lungs or blood vessels and other tissues (not shown) on or close to the heart 904, the ultrasonic waves transmitted by the probe surface 321 are reflected by the heart 904 / lungs, the blood vessels or the other tissues, and are received by the probe surface 321 for the ultrasonic imaging device to generate an ultrasonic image corresponding to the heart 904, the blood vessels or the other tissues. The surgeon can refer to the ultrasonic image thus generated to determine whether the leading tip end 311 will cause damages to the heart 904 / lungs, or whether there are blood vessels or other tissues in the moving direction of the leading tip end 311, and to make proper adjustments in the moving direction of the leading tip end 311. As such, the insertion route may be established to avoid the risks of abrading or puncturing the heart 904 and causing damages to other important blood vessels and tissues. Consequently, risks during Nuss procedure are greatly reduced.

When the leading tip end 311 of the elongated introducer body 31 is led to exit the second incision 906 made at the left lateral side of the patient' s chest 902, the concave steel bar 4 is coupled to the leading tip end 311 by attaching the string 5 that is connected to the concave steel bar 4 to the through hole 316 formed in the leading tip end 311. Subsequently, as the elongated introducer body 31 retrieves the insertion route, the concave steel bar 4 comes next to follow the insertion route passing through and between the heart 904 / lungs and the sternum 903 / ribs without causing any damages. Lastly, when the leading tip end 311 is led to exit the first incision 905 in the patient' s chest 902 such that the elongated introducer body 31 is completely outside of the patient's chest 902, while the concave steel bar 4 is properly disposed inside the patient's chest 902, the string 5 is uncoupled from the leading tip end 311 and the concave steel bar 4. Next, the concave steel bar 4 is flipped by 180 degrees such that the forward-facing surface 41 of the curved middle portion of the concave steel bar 4 faces the heart 904 / lungs and the rearward-facing surface 42 of the curved middle portion faces the sternum 903 / ribs so as to push outward the sternum 903 (as shown in Figure 3) to correct the deformity.

In summary, through the provision of the ultrasonic probe 32 at the leading tip end 311 of the elongated introducer body 31, the introducer device 3 of the present invention helps the surgeon in determining the route to pass the elongated introducer body 31 inside the patient' s chest 902, especially through and between the heart 904 and the sternum 903, in order to establish the insertion route. This way, the risks of abrading and puncturing the heart 904 / lungs, blood vessels, or other important organs and tissues in the patient's chest 902 is significantly reduced as compared to solely depending on the surgeon's clinical experience. Therefore, when the concave steel bar 4 is subsequently coupled to the introducer device 3 for retrieving the insertion route as the introducer device 3 is led to retrieve the same, the concave steel bar 4 is ensured to pass through the patient's chest 902 without damaging the heart 904, blood vessels, or other important organs and tissues in the patient's chest 902.

While the present invention has been described in connection with what are considered the most practical and preferred embodiments, it is understood that this invention is not limited to the disclosed embodiments but is intended to cover various arrangements included within the scope of the broadest interpretation so as to encompass all such modifications and equivalent arrangements.

## Claims

1. An introducer device (3) for use in Nuss procedure to guide a concave steel bar (4) in retrieving an insertion route which passes through and between the sternum (903) / ribs and the heart (904) / lungs of a patient (900), and which is curved away from the sternum (903) / ribs, the concave steel bar (4) having a curved middle portion which is configured to fit in the insertion route, and which has a forward-facing surface (41) and a rearward-facing surface (42) that are concave and convex respectively, said introducer device (3) comprising:
an elongated introducer body (31) which extends in a lengthwise direction to terminate at a leading tip end (311) that is adapted to be coupled to the concave steel bar (4) for retrieving the insertion route, and a grip end (312) that is opposite to said leading tip (311), and which has a curve segment (313) disposed proximate to said leading tip end (311), and configured to establish the insertion route when said leading tip end (311) is led to pass through and between the sternum (903) / ribs and the heart (904) / lungs subsequent to being introduced through a first incision (904) made at a lateral side of the patient's chest (902), said curve segment (313) having a sternum-side surface (314) and a heart-side surface (315) that are concave and convex respectively;
said introducer device (3) being **characterized by** further comprising an ultrasonic probe (32) coupled to said leading tip end (311) and movable along with said leading tip end (311) so as to transmit ultrasonic waves towards the heart (904) / lungs of the patient and to receive reflected ultrasonic waves traveling from the heart (904) /lungs when passing through and between the sternum (903) /ribs and the heart (904) / lungs.

2. The introducer device (3) as claimed in Claim 1, **characterized in that** said ultrasonic probe (32) has a probe surface (321) which is oriented to face the heart (904) / lungs, and which extends in the lengthwise direction from said heart-side surface (315) of said curve segment (313).

3. The introducer device (3) as claimed in Claim 1, **characterized in that** said leading tip end (311) of said elongated introducer body (31) is formed with a through hole (316) that is adapted to be attached to a string (5) connecting the concave steel bar (4) after said leading tip end (311) is led to exit a second incision (906) made at the other lateral side of the patient's chest (905) so as to enable the concave steel bar (4) to pass through and between the heart (904) / lungs and the sternum (903) /ribs as said elongated introducer body (31) retrieves the insertion route.

4. The introducer device (3) as claimed in Claim 1, **characterized in that** said ultrasonic probe (32) is adapted to be connected to an ultrasonic imaging device so as to generate an ultrasonic image in connection with the ultrasonic waves received as a result of the traveling of the reflected ultrasonic waves from the heart (904) / lungs of the patient (900).

## Patentansprüche

1. Einführungsvorrichtung (3) zur Verwendung bei einer Operation nach Nuss, um einen konkaven Stahlbügel (4) beim Wiederherstellen eines Einsetzungsweges zu führen, der zwischen dem Brustbein (903)/den Rippen und dem Herzen (904)/den Lungen eines Patienten (900) hindurchgeht und der von dem Brustbein (903)/den Rippen weg gewölbt ist, wobei der konkave Stahlbügel (4) einen gekrümmten mittleren Abschnitt aufweist, der dafür konfiguriert ist, in den Einsetzungsweg zu passen, und der eine nach vorn zeigende Fläche (41) und eine nach hinten zeigende Fläche (42) aufweist, die jeweils konkav beziehungsweise konvex geformt sind, wobei die Einführungsvorrichtung (3) Folgendes umfasst:
einen länglichen Einführungskorpus (31), der sich in einer Längsrichtung erstreckt, um an einem vorderen Spitzenende (311), das dafür eingerichtet ist, an den konkaven Stahlbügel (4) zum Wiederherstellen des Einsetzungsweges gekoppelt zu werden, und einem Griffende (312), das zu dem vorderen Spitzenende (311) entgegengesetzt ist, zu enden, und der ein Krümmungssegment (313) aufweist, das nahe dem vorderen Spitzenende (311) angeordnet und dafür konfiguriert ist, den Einsetzungsweg herzustellen, wenn das vordere Spitzenende (311) geführt wird, um zwischen dem Brustbein (903)/den Rippen und dem Herzen (904)/den Lungen hindurchzugehen, nachdem es durch einen ersten, an einer seitlichen Seite des Brustkorbes (902) des Patienten vorgenommenen, Einschnitt (904) eingeführt wurde, wobei das Krümmungssegment (313) eine brustbeinseitige Fläche (314) und eine herzseitige Fläche (315) aufweist, die jeweils konkav beziehungsweise konvex sind,
wobei die Einführungsvorrichtung (3) **dadurch gekennzeichnet ist, dass** sie ferner eine Ultraschallsonde (32) umfasst, die an das vordere Spitzenende (311) gekoppelt ist und zusammen mit dem vorderen Spitzenende (311) bewegt werden kann, um so Ultraschallwellen zu dem Herzen (904)/den Lungen des Patienten hin zu übertragen und reflektierte Ultraschallwellen zu empfangen, die sich von dem Herzen (904)/den Lungen aus bewegen, wenn sie zwischen dem Brustbein (903)/den Rippen und dem Herzen (904)/den Lungen hindurchgeht.

2. Einführungsvorrichtung (3) nach Anspruch 1, **dadurch gekennzeichnet, dass** die Ultraschallsonde (32) eine Sondenfläche (321) aufweist, die so ausgerichtet ist, dass sie dem Herzen (904)/den Lungen gegenüberliegt, und die sich in der Längsrichtung von der herzseitigen Fläche (315) des Krümmungssegmentes (313) aus erstreckt.

3. Einführungsvorrichtung (3) nach Anspruch 1, **dadurch gekennzeichnet, dass** das vordere Spitzenende (311) des länglichen Einführungskorpus (31) mit einem Durchgangsloch (316) geformt ist, das dafür eingerichtet ist, an einem Strang (5) befestigt zu werden, der mit dem konkaven Stahlbügel (4) verbindet, nachdem das vordere Spitzenende (311) so geführt worden ist, dass es aus einem zweiten, an der anderen seitlichen Seite des Brustkorbs (902) des Patienten vorgenommenen, Einschnitt (905) austritt, um so zu ermöglichen, dass der konkave Stahlbügel (4) zwischen dem Herzen (904)/den Lungen und dem Brustbein (903)/den Rippen hindurchgeht, wenn der längliche Einführungskorpus (31) den Einsetzungsweg wiederherstellt.

4. Einführungsvorrichtung (3) nach Anspruch 1, **dadurch gekennzeichnet, dass** die Ultraschallsonde (32) dafür eingerichtet ist, mit einer Ultraschall-Bildgebungseinrichtung verbunden zu werden, um so in Verbindung mit den Ultraschallwellen, die als Ergebnis der Bewegung der von dem Herzen (904)/den Lungen des Patienten (900) reflektierten Ultraschallwellen empfangen werden, ein Ultraschallbild zu erzeugen.

## Revendications

1. Dispositif introducteur (3) destiné à être utilisé dans une procédure de Nuss pour guider une barre en acier concave (4) afin de l'aider à retrouver une voie d'insertion qui passe à travers et entre le sternum (903)/les côtes et le coeur (904)/les poumons d'un patient (900), et qui est incurvé dans une direction opposée au sternum (903)/aux côtes, la barre en acier concave (4) ayant une partie intermédiaire incurvée qui est configurée pour s'ajuster dans la voie d'insertion, et qui a une surface tournée vers l'avant (41) et une surface tournée vers l'arrière (42) qui sont concave et convexe, respectivement, ledit dispositif introducteur (3) comprenant :
un corps d'introducteur allongé (31) qui s'étend dans un sens de la longueur pour se terminer au niveau d'une extrémité de pointe avant (311) qui est conçue pour être couplée à la barre en acier concave (4) pour retrouver la voie d'insertion, et une extrémité de préhension (312) qui est opposée à ladite pointe avant (311), et qui a un segment incurvé (313) disposé à proximité de ladite extrémité de pointe avant (311), et configurée pour établir la voie d'insertion lorsque ladite extrémité de pointe avant (311) est amenée à passer à travers et entre le sternum (903)/les côtes et le coeur (904)/les poumons après avoir été introduite dans une première incision (904) située sur un côté latéral du thorax (902) du patient, ledit segment incurvé (313) ayant une surface côté sternum (314) et une surface côté coeur (315) qui sont concave et convexe, respectivement ;
ledit dispositif introducteur (3) étant **caractérisé en ce qu'**il comprend en outre une sonde ultrasonore (32) couplée à ladite extrémité de pointe avant (311) et amovible conjointement à ladite extrémité de pointe avant (311) de manière à transmettre les ondes ultrasonores en direction du coeur (904)/des poumons du patient et de manière à recevoir des ondes ultrasonores réfléchies se propageant depuis le coeur (904)/les poumons lorsqu'elles passent à travers et entre le sternum (903)/les côtes et le coeur (904)/les poumons.

2. Dispositif introducteur (3) selon la revendication 1, **caractérisé en ce que** ladite sonde ultrasonore (32) a une surface de sonde (321) qui est orientée pour faire face au coeur (904)/aux poumons, et qui s'étend dans le sens de la longueur depuis ladite surface côté coeur (315) dudit segment incurvé (313).

3. Dispositif introducteur (3) selon la revendication 1, **caractérisé en ce que** ladite extrémité de pointe avant (311) dudit corps d'introducteur allongé (31) est formée avec un trou débouchant (316) qui est conçu pour être fixé à une ficelle (5) raccordant la barre en acier concave (4) après que ladite extrémité de pointe avant (311) est amenée à sortir par une seconde incision (906) située au niveau de l'autre côté latéral du thorax (905) du patient de manière à permettre que la barre en acier concave (4) passe à travers et entre le coeur (904)/les poumons et le sternum (903)/les côtes alors que ledit corps d'introducteur allongé (31) retrouve la voie d'insertion.

4. Dispositif introducteur (3) selon la revendication 1, **caractérisé en ce que** ladite sonde ultrasonore (32) est conçue pour être raccordée à un dispositif d'imagerie ultrasonore afin de générer une image ultrasonore relativement aux ondes ultrasonores reçues en résultat de la propagation des ondes ultrasonores réfléchies du coeur (904)/des poumons du patient (900).
